# EUROPEAN PATENT SPECIFICATION

(11) **EP 0 561 903 B1**
(45) Date of publication and mention of the grant of the patent: **05.07.1995**
(21) Application number: 92900726.8
(22) Date of filing: 18.11.1991
(51) Int. Cl.: A61B 17/36

(54) **A device for interstitial laser energy delivery**
Vorrichtung zur interstitiellen Applikation von Laserlicht
Dispositif pour l'application interstitielle d'énergie à laser

(30) Priority: 10.12.1990 US 625332
(43) Date of publication of application: 29.09.1993
(73) Proprietor: HOWMEDICA INC., New York New York 10017 (US)
(72) Inventor: MAKOWER, Joshua, Nanuet, NY 10954 (US)
(74) Representative: Moore, James William, Dr.
(86) International application number: US9108388
(87) International publication number: WO9210142

(56) References cited:
- DE-A- 2 826 383
- DE-A- 3 840 749
- US-A- 4 760 840
- US-A- 4 950 267
- US-A- 4 955 882

## Description

### Field of the Invention

This invention relates to a apparatus and method for laser energy delivery to internal organs for treatment thereof and in particular, treatment of diseases of the prostate, including benign prostatic hypertrophy (BPH) and prostatic cancer. More particularly, the invention relates to a catheter including a needle means for delivering a laser guiding optical fiber directly to the area to be treated by energy delivery.

### Background of the Invention

Prostatic disease is one of the most common diseases in men in the United States. Prostatic disease, as referred to here, includes benign prostatic hypertrophy (BPH) and prostatic cancer. These two etiologies affect a majority of men over the age of 60.

The clinical symptoms of BPH include urinary tract outlet obstruction due to an enlarged prostate. The etiology of BPH, while not fully understood, has focused on two hypotheses. The first hypothesis has identified the hyperplastic cell morphology as a stromal cell disease. The second hypothesis has investigated the effects of prohormone dihydrotestosterone (DHT), which is the primary mediator of androgen action in the prostatic cells.

The currently accepted treatment for BPH is transurethral resection of the prostate (TURP). Approximately 300,000 TURPs per year are performed to treat this disorder in the United States. Morbidity and mortality for TURP are 17 and 1 percent, respectively, for all age groups combined. Higher complication rates occurs in older populations with an annual surgical and hospitalization cost in excess of $1 billion per year.

Among other treatment available for the condition of BPH are pharmacological means such as vasoactive and antiandrogen agents. The vasoactive drugs primarily used are block alpha₁ receptors, without effecting the alpha₂, receptors. These drugs reduce smooth muscle tone within the prostate, which is in part responsible for the mechanical obstruction of urine through the prostatic urethra. Data on this treatment suggest good efficacy in relieving symptoms, but it should be noted that mechanical obstruction may still exist and may promote the development of urinary tract infection bladder stones and possible upper urinary tract obstruction.

Antiandrogen agents have also been used to reduce the symptoms associated with BPH. The primary function of these antiandrogen blockers is to reduce the effects of DHT activity in the prostate by competing for the androgen receptors. While there has been evidence of the clinical efficacy of these agents in reducing the size of the prostate and relieving the symptoms associated, the problem with this pharmacological intervention has been the slow onset of therapeutic action.

With regard to prostatic cancer, both the incidence ad mortality are on the rise. It is expected that over 100,000 new cases will be diagnosed this year alone, with some 30,000 cases proving fatal.

While the etiology of prostatic cancer is also not well known, it has been suggested that this disease is either biochemically or genetically induced. The symptoms of prostatic cancer are insidious and are usually not clinically manifest until the course of the disease is far advanced. The current treatment of choice for prostatic cancer is to perform a radical prostatectomy which involves surgical excision of the prostate gland.

In both cancerous and benign conditions, the cause of the reduction of the available flow channel in the lumen of the vessel i.e., the prostatic urethra, is an externally induced compression of the vessel wall due to the proliferation of epithelial, prostatic cell tissue. In order to be effective, treatment of diseases of the prostate must cause a reduction in the mass of the prostatic tissue responsible for creating the compressive forces on the urethra which results in the obstruction of flow through the lumen of the urethra. This is accomplished either by surgical excision of the tissue or by other means which will cause necrosis of the cells and shrinkage of the tissue mass.

Laser energy may be applied at at least four different levels in order to affect the tissue being treated. The first and lowest level of laser energy delivery is induced fluorescence. At this level, laser light energy is directed into the cells to reversibly energize the cells. The fluorescence effect occurs as energy is given off in returning to the lower energy state. The next level results in cellular change due to photo-effect. Laser light energy is directed into the cells at an irreversible level, but below that required to produce hyperthermia. At this level of energy delivery, cellular change (including necrosis if desired) occurs at the molecular level due to the photo effects of laser light. The third level of energy delivery results in hyperthermia by raising cells to a temperature level (42-44°C) where necrosis occurs. The fourth level of energy delivery is vaporization. This requires delivery of laser energy sufficient to produce temperatures of about 100°C in the tissue being treated.

Laser hyperthermia has been suggested as a possible means for necrosis of diseased prostatic cells. But, to date, practical means for applying laser hyperthermia have not been developed.

U.S. patent No. 4,672,963 to Barken discloses an apparatus and method for laser surgery which uses a computer controlled, ultrasonic imaging system to position a laser light guide within a patient. This patent discusses prostatic disease and purports to provide suitable apparatus for treatment; however, no specific means for directing the laser energy to the area in need of treatment is disclosed. Barken states only that an optical light guide can be inserted into the body through a relatively small surgical opening.

U.S. Patent No. 4,950,267 to Ishihara et al. discloses a laser beam treatment device for an endoscope. The endoscope delivers a laser probe to a position in a body, from which the laser probe is thrust into the part of the organ to be treated. The disclosure is not specific as to how the laser probe is inserted into the organ. Known fiber optic elements generally do not posses sufficient rigidity to mechanically puncture a body structure such as the urethral wall. Furthermore, Ishihara discloses a number of alternative laser probes having blunt or rounded distal ends. It is unlikely that these probes could be mechanically forced into an organ even if they possessed substantial structural rigidity. Thus, it appears that insertion of at least some of the Ishihara laser probes requires coincidental application of laser energy to burn a hole into the organ. This method of insertion is undesirable because, upon withdrawal of the instrument, a small hole will remain with the possibility of abscess and infection.

German patent application DE-A-28 26 383 discloses a tubular probe comprising a needle in which is slideably disposed an optical fiber for guiding a laser beam for laser-surgical applications wherein the probe is directly placed against, or inserted into, the tissue to be treated.

The above-mentioned citations discuss only laser hyperthermia. They do not address possible treatments using the other three energy levels previously discussed. Thus, there is a need in the field of medical laser energy delivery, including treatment of prostatic disease, for an effective means for delivering various laser energy levels to the areas to be treated. In the treatment of prostatic disease, alternatives are needed to the more radical and consequently complicated and more dangerous surgical procedures which currently are the treatments of choice, namely, TURP for BPH and radical prostatectomy for prostatic cancer.

### Summary of the Invention

It is therefore a object of the present invention to provide a practical means for laser energy delivery to the prostate or other organs adjacent to a body passageway.

It is also an object of the present invention to provide a procedure for treatment of the prostate or other organs which is potentially bloodless compared to known procedures and is a less invasive single procedure that can be performed on an outpatient basis. According to the present invention, these and other objects are achieved by providing a catheter with at least one moveable hollow needle that carries a fiber optic element. In this manner, the catheter may be inserted into a body passageway adjacent to the organ to be treated. The moveable needles are extendable to mechanically puncture the passageway wall and carry the fiber optic elements into the organ to the area to be treated. After the desired level of laser energy has been applied to the area being treated, the fiber optic elements and hollow needles are withdrawn into the catheter. The catheter is then removed from the passageway. Thus, laser energy delivery with the present invention leaves behind only small puncture wounds in the passageway wall. Due to the nature of the mechanical puncture wounds they are capable of healing quickly with a minimum risk of infection.

The present invention generally includes the following components: At least one fiber optic element for delivering laser light from a laser energy source to the area of the prostate to be treated. The fiber optic element is slidably received in and carried by a hollow needle. The hollow needle has a sharpened distal end in order to easily mechanically puncture the passageway wall and enter the organ to carry the fiber optic element to the area to be treated. The fiber optic element is slidably disposed within the needle to allow relative axial movement between the element and the needle. Thus, the needle is retractable separately from the fiber optic element.

The needle is received in a flexible catheter shaft which is inserted into the passageway. Any number of needles may be carried in the catheter shaft. The number of needles is limited only by the diameter of the catheter shaft. Generally, one fiber optic element is carried by each needle. The needles are slidably received in needle lumens within the catheter shaft. At the distal end of the catheter shaft a distal tip is provided to guide the needles outward and into the prostate. The means for directing the needles may be simply a number of curved channels communicating with the needle lumens to guide the needles in the desired direction, or may comprise a bearing and track system for guiding each needle.

The present invention also includes a means for actuating and controlling the needles in order to move each needle between a first position, sheathed within the catheter shaft and distal tip, and a second position, extending out of the distal tip and into the organ. The actuation and control means is capable of moving each needle between the first and second positions, both independent of the fiber optic element and in conjunction with the fiber optic element. By using a suction-irrigation device with the hollow needle, the hollow needle may serve as a vehicle to aspirate or irrigate the area receiving laser treatment, or the needle may be completely withdrawn into the catheter shaft. A suction and irrigation device may also be used in communication with the needle receiving lumen in the catheter shaft to provide suction or irrigation in the passageway at the point where the fiber optic element enters the wall.

To support the needle during puncturing and insertion into the passageway wall means for temporarily fixing the catheter in the passageway is provided. This means comprises at least one inflatable dilation balloon surrounding at least part of the distal end of the catheter shaft and distal tip. A lumen is provided in the catheter shaft to direct an inflation fluid to the dilation balloon.

In one alternative embodiment the needle is provided with a steering fiber in order to guide the needle once it has entered the prostate. Tension induced in the steering fiber causes the needle to bend and thus change direction.

Thus the invention provides
an apparatus for laser energy delivery to an organ (43) adjacent to a body passageway (42) defined by a passageway wall, wherein said apparatus includes:
at least one fiber optic element (23) for delivering laser light from a laser energy source (11) to an area of the organ (43) to be treated;
at least one needle means (15) for mechanically puncturing the passageway wall and organ (43);
needle and fiber optic element delivery means (1) for delivering each of said at least one needle means (15) and each of said at least one fiber optic element (23) to a position in the passageway (42) adjacent to the organ (43) to be treated;
said needle and fiber optic element delivery means (1) is a tubular body (2), having an outer periphery, a distal end (4) and a proximal end (5), and defines at least one needle lumen (16) corresponding to each of said at least one needle means (15), for slideably receiving said at least one needle means (15); and
said needle and fiber optic element delivery means (1) further includes at least one needle direction means (17, 18) for directing said at least one needle means (15) into the organ (43), wherein said needle directing means (17, 18) includes at least one curved needle director channel (17), such that one said curved needle director channel (17) communicates with each of said at least one needle lumens (16), and has an outlet (18) on the periphery of said tubular body (2) opening onto said body passageway (42); and
each of said at least one needle means (15), for mechanically puncturing the passageway wall and organ (43), includes a flexible tubular shaft (33) and a sharpened distal needle tip (34), joined at joint (35) to said shaft (33);
characterized in that:
said at least one fiber optic element (23) has an unobstructed distal end;
each of said at least one needle means (15) is hollow, and defines a lumen (32) therein, with an unobstructed distal opening (44), said lumen (32) being for carrying one of said at least one fiber optic element (23) through the puncture to the area of the organ (43) to be treated; and
each of said at least one fiber optic element (23) is slideably disposed within one of said at least one needle means (15) to allow relative axial movement between said at least one fiber optic element (23) and said at least one needle means (15), such that said at least one fiber optic element (23) is extendable out of said distal opening (44) of said lumen (32), with each said at least one needle means (15) being separately retractable from the said fiber optic element (23) slideably disposed therein, to expose said unobstructed distal end of said fiber optic element (23) to the area to be treated.

### Brief Description of the Drawings

The features and advantages of the invention will be more readily apparent from the following detailed description of the preferred embodiments, illustrated in the drawing figures, wherein:
FIG. 1 is a schematic view of the apparatus of the present invention showing the catheter and ancillary devices;
FIG. 2 is a section view of the catheter tip of a preferred embodiment of the apparatus of the present invention;
FIG. 3 is a detailed view of a portion of FIG. 2 showing the needle and needle lumen;
FIG. 4 is a section view of the catheter tip of an alternative preferred embodiment of the apparatus of the present invention;
FIG. 5 is a section view of an alternative embodiment of the distal tip and needle director channels according to the present invention;
FIGS. 6a and 6b are cross sectional views of the tip shown in FIG. 5, taken along lines 6a-6a and 6b-6b, respectively;
FIG. 7 is a section view of a preferred embodiment of a needle used in the apparatus of the present invention;
FIG. 8 is a section view of a steerable needle used in the apparatus of the present invention;
FIG. 9 is a cross sectional view of the needle of FIG. 8, taken along line 9-9;
FIG. 10 is a section view of the catheter tip according to an alternative preferred embodiment of the apparatus of the present invention; and
FIG. 11 is a section view of the catheter tip of a preferred embodiment of the apparatus of the present invention shown in transurethral insertion made with the needles and fiber optic element in various stages of deployment in prostatic tissue.

### Detailed Description of the Preferred Embodiments

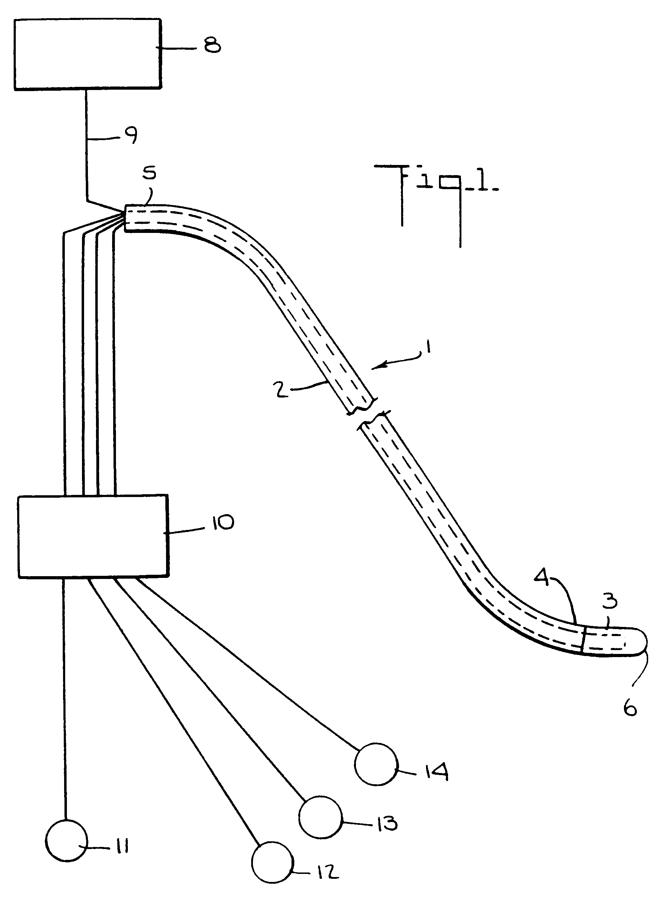

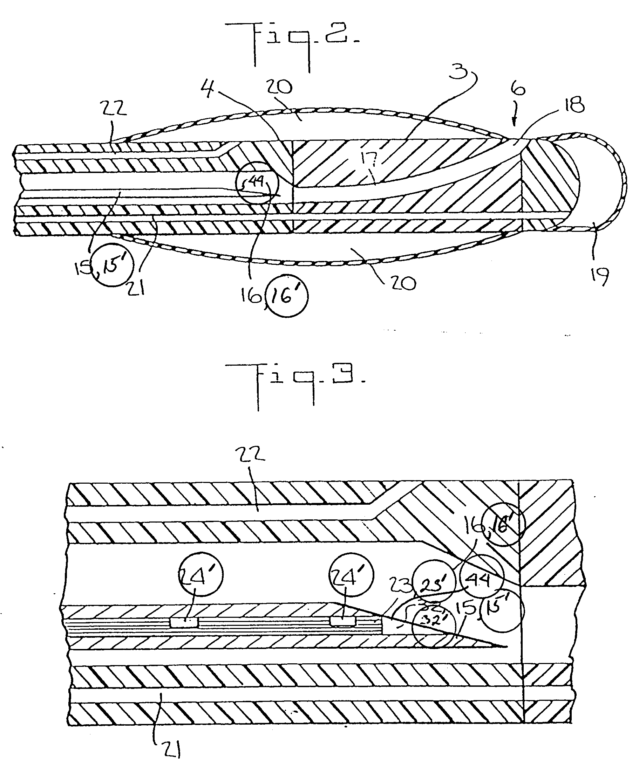

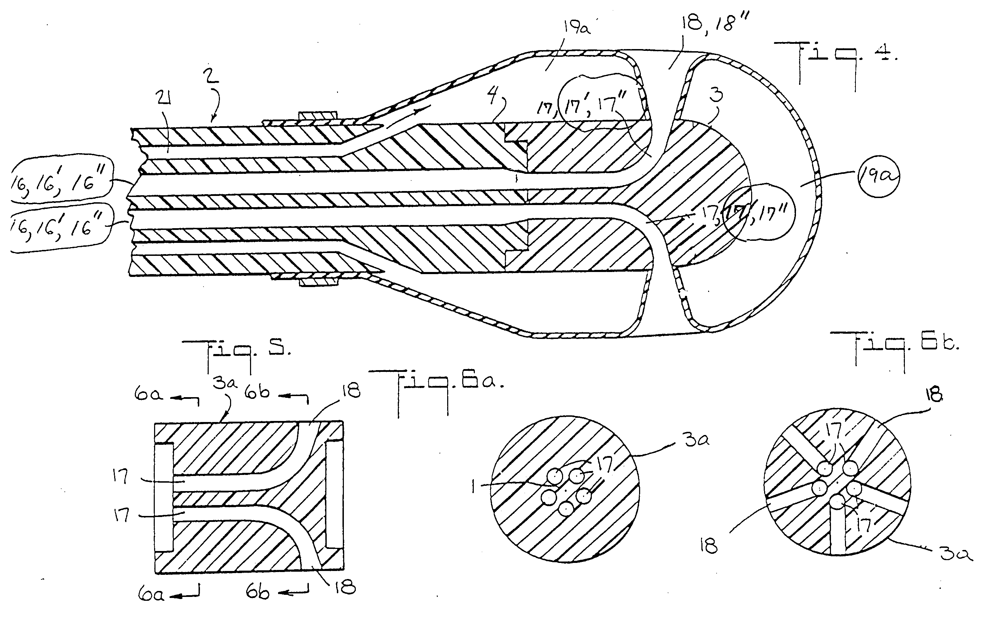

Shown generally in FIG. 1, the present invention includes catheter 1, which delivers a needle system (shown in detail in FIGS. 3 and 7-9) that places one or more fiber optic elements and thermo-measuring devices through the urethral or rectal wall and into the bulk of the prostate mass. Laser energy is then delivered by the fiber optic element to hyperthermally treat selected areas. For the purposes of clarity and conciseness, the detailed description is made with reference only to the urethra, rectum and prostate. This is not intended to be limiting of the present invention, as it will be readily appreciated that the present invention is useful for laser energy delivery to any organ or body part adjacent to a passageway accessible by a catheter.

Catheter 1 has a semi-rigid or flexible shaft 2 ending at distal tip 3 and beginning at proximal end 5. The diameter of catheter 1 may be small enough to allow it to be inserted down the shaft of a known urethroscope, proctoscope or resectoscope. Alternatively, catheter 1 may be much larger without departing from the scope of the invention; and, thus be inserted directly into the rectum or urethra.

Needle control cable 9 extends from proximal end 5 and connects the needle system to an external needle actuation and control means 8. In practice, the configuration of needle control cable 9 will vary depending on the particular embodiment of the invention used. These variations will be apparent to persons skilled in the art based on the description of the alternative embodiments below.

In addition to needle actuation and control means 8, one or more of the following devices are used to assist in positioning and operating the invention: laser energy source 11, suction-irrigation device 12, thermo-monitoring device 13, and fiberoptic visualization apparatus 14. These devices cooperate with catheter 1 through multi-device interface 10. The operation and function of each of these known devices should be understood by those skilled in the art. The cooperation of each device with the present invention will be better understood after the various components of the present invention have been described in greater detail below.

FIG. 2 illustrates one preferred embodiment of the needle system and distal tip 3 of the present invention. Distal tip 3 is secured to the distal end 4 of catheter shaft 2. Needle 15 lies in needle lumen 16 within shaft 2. A long, spinal-type needle with sufficient flexibility to follow the urethral or rectal passageway is shown. Any similar long, fine needle may be used. Needle director channel 17 is provided in distal tip 3, in communication with needle lumen 16. Director channel 17 opens on the periphery of distal tip 3, through needle outlet 18.

When catheter 1 has been placed within the urethra or rectum, with distal tip 3 adjacent to the prostate, needle 15 may be advanced by actuation and control means 8. In practice, various actuation means are possible. A person of ordinary skill in the art will recognize hydraulic, spring loaded, mechanical translational or rotational cable, shape memory alloy, or electro-magnetic mechanisms as useful for this purpose. When advanced, needle 15 extends from lumen 16 into director channel 17 and is thus curved outward, toward the urethral or rectal wall and into the prostate.

The position of catheter 1 and extension of needle 15 may be observed using various visualization means such as fluoroscopy, magnetic resonance spectroscopy, proctoscopy, urethroscopy, transrectal ultrasound, and transurethral ultrasound. Specific targeting of certain types of small lesions in the prostate (i.e., prostate cancer) will require the use of interstitial guidance such as ultrasound, MRI or fluoroscopy. More massive benign conditions, such as BPH, may not require such guidance.

To maintain the position of the catheter and dilate the urethra or rectum (compress the prostate) while needle 15 is entering the prostate, one or more balloons may be used. In the embodiment shown in FIG. 2, shaft balloon 20 and tip balloon 19 are used for these purposes. An inflation substance such as saline or air is delivered through lumens 22 and 21, respectively. By pressing against the passageway wall, the balloons fix the catheter to provide a firm base for supporting the needle as it punctures and enters the prostate.

Additionally, the effectiveness of the laser energy delivery can be increased by restricting blood flow to the tissue cells to be necrosed. For example, effective laser hyperthermia requires that the temperature of the target cells be raised to a minimum of about 42.5°C and maintained at that temperature for a specified period of time. Blood supply to the diseased target cells acts as a heat sink that absorbs thermal energy and prevents those cells from being heated sufficiently. The heat sink effect prevents the target cells from reaching the desired temperature without raising the temperature of surrounding normal tissue cells high enough to cause damage. The dilation balloon(s) press against the prostate to restrict blood flow to the target cells and reduce the unwanted heat sink effect.

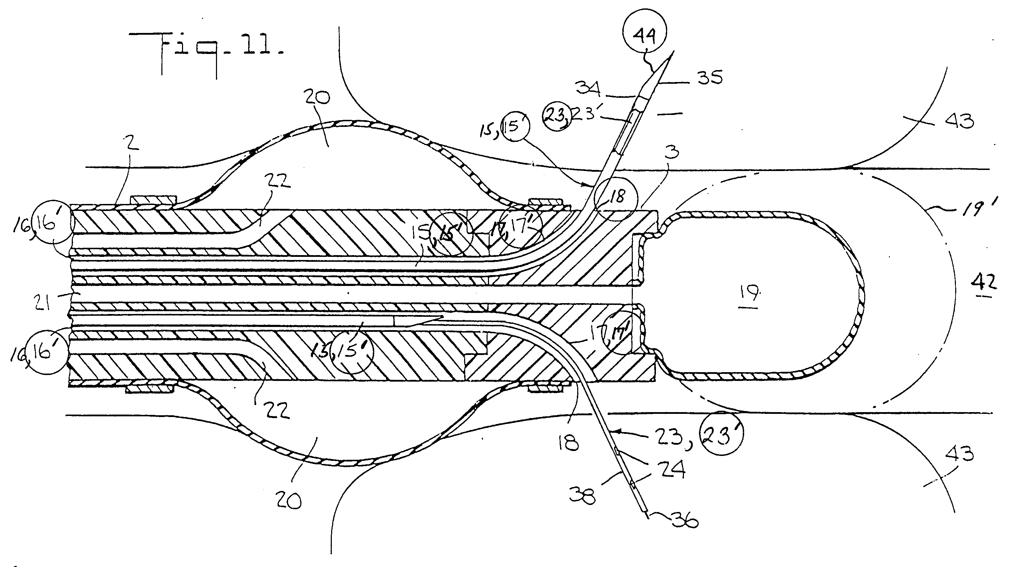

The details of needle 15 are best seen in
FIG 3. Fiber optic element 23 is slideably received in lumen 32 in the needle. In a preferred embodiment, fiber optic element 23 is simply a bare optical fiber. Modifications such as cladding and shaped ends may be used (see FIG. 8). One or more thermo-measuring devices are attached to element 23 to monitor the temperature at the energy delivery location. For this purpose two thermocouples 24 are shown in FIG. 3. Fiber optic thermo-measuring devices also may be used. The leads for thermocouples 24 are attached to fiber optic element, 23 and lie within lumen 32. Lumen 32 also may be used for aspiration or irrigation.

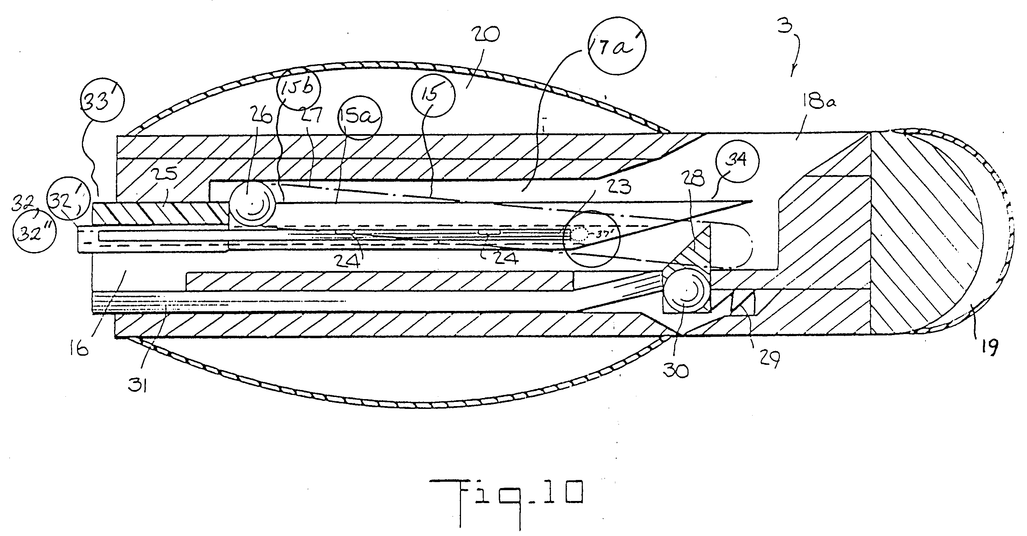

FIG. 7 illustrates a possible alternative embodiment for needle 15. Instead of being formed as a single piece, the needle shown in FIG. 7 has a sharpened needle tip 34 joined to a flexible needle shaft 33 by joint 35. Tip 34 is made of medical grade stainless steel and sharpened to a razor-like edge. Shaft 33, as are all other components, is made of standard bio-compatible plastic which may be selected by those of ordinary skill in the art. Again, central lumen 32 is provided within the needle to carry the fiber optic element and allow for aspiration or irrigation.

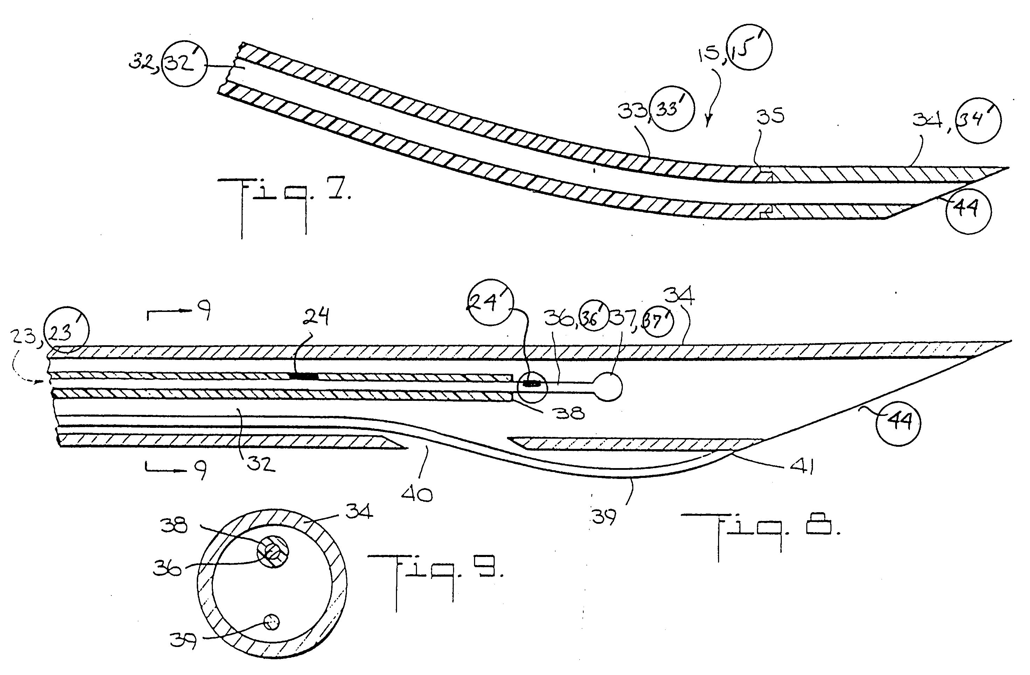

FIG. 4 illustrates an alternative embodiment of the present invention wherein the dilation and fixation means is formed as a single balloon 19a surrounding distal tip 3 and proximal end 4 of catheter shaft 2. Needle outlets 18 are provided in balloon 19a to allow the needles to pass from director channels 17 into the prostate. Balloon 19a is inflated by one or more fluid lumens 21. This configuration concentrates the blood restricting and supporting effects of balloon 19a around the point of entry of the needles into the prostate.

Distal tip 3, shown in FIG. 1, is provided with a single needle director channel 17 for a single needle. It will be readily appreciated that multiple needles may be utilized, with the number being restricted only by the diameter of catheter 1. FIGS. 5 and 6 illustrate an alternative embodiment of a distal tip 3a having five director channels 17 to accommodate five separate needles. The five separate director channels 17 alternatively may communicate with a single large lumen in catheter shaft 2 or director channels 17 may individually communicate with five separate needle lumens provided in catheter shaft 2.

Referring to FIGS. 8 and 9, a steerable needle according to the present invention may be described. In this embodiment, a needle steering fiber 39 is provided within lumen 32. Steering fiber 39 exits the needle through opening 40 near the needle tip. Steering fiber 39 is fixed to the needle adjacent the sharpened tip at joint 41. Tension induced in steering fiber 39 causes the needle to bend due to the eccentric positioning of the steering fiber within lumen 32. This bending allows the surgeon to steer the needle to the precise spot where hyperthermia is required, after the needle has punctured and entered the prostate.

FIG. 8 also illustrates a possible embodiment of fiber optic element 23. As shown in FIG. 8, optical fiber 36 is provided with cladding 38 and spherical end 37 in order to dissipate the laser energy over a wider angle at the point of delivery. Cladding 38 is standard, commercially available optical cladding.

FIG. 10 shows a further alternative embodiment of the catheter distal tip 3 according to the invention. In this embodiment, needle 15 is advanced forward by manipulation of cable 25, attached at the proximal end of needle 15. The correct movement of needle 15 is ensured by bearing 26, which moves with needle 15 and rides smoothly in track 27. Needle port 18a may have a breakthrough covering or may be exposed as shown.

In order to finally position needle 15, catch 28 is retracted by cable 31 and bearing 26 is pushed past the catch. Cable 31 is then released and compression spring 29 causes catch 28 to hold needle 15 in place due to its biasing action around pivot 30. Thus, catch 28 adds both directional assistance and provides resistance to maintain needle 15 in position. The needle may be definitively locked in position by locking cable 31. Spring 29 may be a plastic or metal spring.

Attached at the base of needle 15 is an aspiration/irrigation lumen 32 which allows the needle to introduce the fiber optic element 23 (with thermocouples 24) and act as an irrigation or suction vehicle.

The overall operation of the present invention may be explained by reference to FIG. 11. Catheter 1 is inserted into the urethra 42 (or rectum) and placed adjacent to the area of the prostate 43 where delivery of laser energy is desired. Shaft balloon 20 and tip balloon 19 (inflated at 19') are inflated as required to secure the position of catheter 1 and compress the prostate. As shown in FIG. 11, catheter 1 employs distal tip 3a (FIG. 5) and needles 15 are formed in two parts as shown in FIG. 7.

FIG. 11 shows the present invention at two different stages of operation. In the top half of FIG. 11, needle 15' has just been extended to puncture the urethral wall and enter the prostate 43. As needle 15' is moved into the prostate, it carries with it fiber optic element 23', with thermocouples 24 attached. Once the needle 15' has moved to the desired location in the prostate, the needle may be either withdrawn leaving the fiber optic element 23' in place, or remain in position to act as a irrigation or suction vehicle. If vaporization is to be employed, aspiration through the needle is necessary to remove gasses produced. Control and actuation means 8 is used to control the position of the fiber optic elements with respect to the needles and to control the position of both with respect to the catheter. If necessary, irrigation or aspiration may be conducted through needle lumen 16, director channel 17 and needle outlet 18 which opens into the urethra 42 (or rectum). Aspiration or irrigation for both the needle and the catheter is provided by suction-irrigation device 12.

As shown in the bottom half of FIG. 11, needle 15'' has been withdrawn into needle lumen 16, leaving fiber optic element 23'' in place in the prostate 43. In this embodiment, fiber optic element 23'' comprises optical fiber 36 surrounded by cladding 38. Optical fiber 36 has a plain end, and thermocouples 24 are mounted in cladding 38. Laser energy is provided to the fiber optic elements from laser energy source 11 by known means. Thermocouples 24 are connected to thermo-monitoring device 13, which monitor the temperature of the area being treated in order to precisely control the energy delivery.

After the delivery of laser energy is complete, the fiber optic elements may be repositioned by further extension of the associated needles or by withdrawing the elements and repositioning catheter 1. Alternatively, if a steerable needle is used (such as shown in FIG. 8), the needle may be significantly repositioned without moving catheter 1. After energy delivery is complete, all needles and fiber optic elements are withdrawn and catheter 1 is completely withdrawn from the body.

The structure of the present invention also promotes recovery for the patient after the procedure is complete. Due to deep ablation of the tissue and the mode of delivery, only small needle-size puncture holes remain in the urethra. These will heal quickly, leaving the remaining deep necrotic tissue to sluff and be absorbed subepithelially. Any liquefied or vaporized materials may be suctioned while the needles are still in place. No drainage catheter is needed postoperatively and the procedure may be done on a one day surgery basis.

## Claims

1. An apparatus for laser energy delivery to an organ (43) adjacent to a body passageway (42) defined by a passageway wall, wherein said apparatus includes:
at least one fiber optic element (23) for delivering laser light from a laser energy source (11) to an area of the organ (43) to be treated;
at least one needle means (15) for mechanically puncturing the passageway wall and organ (43);
needle and fiber optic element delivery means (1) for delivering each of said at least one needle means (15) and each of said at least one fiber optic element (23) to a position in the passageway (42) adjacent to the organ (43) to be treated;
said needle and fiber optic element delivery means (1) is a tubular body (2), having an outer periphery, a distal end (4) and a proximal end (5), and defines at least one needle lumen (16) corresponding to each of said at least one needle means (15), for slideably receiving said at least one needle means (15);
said needle and fiber optic element delivery means (1) further includes at least one needle direction means (17, 18) for directing said at least one needle means (15) into the organ (43), wherein said needle directing means (17, 18) includes at least one curved needle director channel (17), such that one said curved needle director channel (17) communicates with each of said at least one needle lumens (16), and has an outlet (18) on the periphery of said tubular body (2) opening onto said body passageway (42); and
each of said at least one needle means (15), for mechanically puncturing the passageway wall and organ (43), includes a flexible tubular shaft (33) and a sharpened distal needle tip (34), joined at joint (35) to said shaft (33)
characterized in that:
said at least one fiber optic element (23) has an unobstructed distal end;
each of said at least one needle means (15) is hollow, and defines a lumen (32) therein, with an unobstructed distal opening (44), said lumen (32) being for carrying one of said at least one fiber optic element (23) through the puncture to the area of the organ (43) to be treated; and
each of said at least one fiber optic element (23) is slideably disposed within one of said at least one needle means (15) to allow relative axial movement between said at least one fiber optic element (23) and said at least one needle means (15), such that said at least one fiber optic element (23) is extendable out of said distal opening (44) of said lumen (32), with each said at least one needle means (15) being separately retractable from the said fiber optic element (23) slideably disposed therein, to expose said unobstructed distal end of said fiber optic element (23) to the area to be treated.

2. The apparatus according to claim 1, still further characterized in that each of said at least one needle means (15) is individually steerable.

3. The apparatus according to claim 2, still further characterized in that each of said at least one needle means (15) further includes:
a peripheral opening (40) in said flexible tubular needle shaft (33), spaced proximally from the distal tip (34) of said needle means (15); and
a steering fiber (39) received in said lumen (32) in each of said at least one needle means (15), and exiting said lumen (32) through said peripheral opening (40), with said steering fiber (39) being joined at joint (41) to said needle means (15) adjacent to its said metal sharpened needle distal tip (34), whereby said needle means (15) is bendable and steerable by inducing tension in said steering fiber (39).

4. The apparatus according to claim 1, still further characterized in that said apparatus further includes suction-irrigation means (12) for alternatively aspirating and irrigating said passageway (42) around the puncture as desired, such that said suction-irrigation means (12) communicates with at least one of: each of said needle lumens (16), and each of said lumens (32) in each of said at least one needle means (15).

5. The apparatus according to claim 1, further characterized in that:
said at least one needle means (15) includes a number of individual tubular shaft needles (15'), with each said individual tubular shaft needle (15') having a flexible shaft (33') and a sharpened distal tip (34') for puncturing said passageway wall and organ (43), and defining a lumen (32') for slideably receiving a corresponding one of an equal number of individual fiber optic elements (23') constituting said at least one fiber optic element (23);
said needle and fiber optic element delivery means (1) is a tubular body (2) having a proximal end (5) and a distal end (4), and defines a plurality of needle lumens (16') equal in number to the number of said individual tubular shaft needles (15'), with one tubular shaft needle (15') being slideably received in each needle lumen (16');
a cylindrical distal tip (3) with an outer periphery is attached to the distal end (4) of the tubular body (2), said distal tip (3) having a plurality of curved needle director channels (17') equal in number to the number of needle lumens (16'), each curved needle director channel (17') communicating with one needle lumen (16') at the distal end (4) of the tubular body (2) and opening at a needle outlet (18') along the periphery of the distal tip (3) to provide means for directing said tubular shaft needles (15') into the organ (43).

6. The apparatus according to claim 1, further including fixation means for temporarily fixing said tubular body (2) and said distal tip (3) in said body passageway (42) relative to said passageway wall;
wherein said fixation means includes:
a first inflatable dilation balloon (20) circumferentially surrounding said tubular body (2);
an inflation lumen (22) provided in said tubular body (2) communicating with said first inflatable dilation balloon (20);
a second inflatable dilation balloon (19) disposed at the distal end of the distal tip (3); and
an inflation lumen (21) provided in said tubular body (2) and extending through said distal tip (3) in communication with the second inflatable dilation balloon (19);
further characterized in that:
said first inflatable dilation balloon (20) is disposed proximally relative to said needle outlets (18) in said distal tip (3); and
said first inflatable dilation balloon (20) and said second inflatable dilation balloon (19) are both provided together on a single apparatus, and are independently capable of being inflated and deflated.

7. The apparatus according to claim 1, still further including fixation means for temporarily fixing the tubular body (2) and distal tip (3) in said body passageway (42) relative to said passageway wall, wherein said fixation means includes:
an inflatable dilation balloon (19a);
an inflation lumen (21) defined by said tubular body (2) and communicating with said inflatable dilation balloon (19a);
still further characterized in that:
said inflatable dilation balloon (19a) surrounds said distal tip (3) and at least a portion of said distal end (4) of said tubular body (2); and
said inflatable dilation balloon (19a) has a number of needle channels (17'') equal to the number of needle lumens (16''), through which said needles are passable, with each said needle channel (17'') being aligned with and communicating with one of said needle outlets (18'').

8. The apparatus according to claim 1, further characterized in that each of said at least one fiber optic elements (23) includes an optical fiber (36), surrounded by optical cladding (38), with a thermo-measuring device (24) provided coaxially with said optical fiber (36) on said optical cladding (38).

9. The apparatus according to claim 8, still further characterized in that said optical fiber (36) has a spherical distal end (37).

10. The apparatus according to claim 1, further characterized in that said fiber optic element (23) is a bare optical fiber with at least one thermo-measuring device (24') provided thereon.

11. The apparatus according to claim 10, still further characterized in that said fiber optic element (23) has a spherical distal end (37').

12. The apparatus according to claim 1, further characterized by the inclusion of needle actuation and control means (8) for individually actuating and controlling said at least one needle lumen (16) to move said needle means (15) between a first position, sheathed within said at least one needle lumen (16), and a second position extending out of said tubular body (2) and into said organ (43) to be treated, said needle actuation and control means (8) being capable of moving said at least one needle means (15) between said first and second positions, alternatively, independently of said at least one fiber optic element (23), and in conjunction with said at least one fiber optic element (23), whereby said at least one fiber optic element (23) and said at least one needle means (15) are capable of being independently selectively positioned and repositioned in said organ (43), as required.

13. The apparatus according to claim 12, still further characterized in that actuation of said at least one needle means (15) is by means selected from the group consisting of hydraulic, spring loaded, mechanical translational cable, mechanical rotational cable, electromagnetic, and shape memory alloy.

14. The apparatus according to claim 1, further characterized in that:
each of said at least one needle means (15) includes a rigid distal needle portion (15a) having a proximal end (15b) and a sharpened distal end (34), said rigid distal needle portion (15a) defining a lumen (32') for slideably carrying said fiber optic element; and
said needle and fiber optic element delivery means (1) further includes a tubular body (2) defining at least one needle lumen (16); and
said apparatus further includes:
a bearing (26) mounted on said rigid distal needle portion (15a) at its said proximal end (15b);
at least one flexible control and actuation cable (25) attached to said proximal end (15b) of said rigid portion (15a) of each of said at least one needle means (15), and slideably disposed within said at least one needle lumen (16) of said tubular body (2);
a structure (33'), attached to said proximal end (15b) of said rigid distal needle portion (15a), and defining at least one lumen (32''), said lumen (32'') communicating with said lumen (32'), slideably carrying one of said at least one fiber optic elements (23), and providing means for aspirating and irrigating the area of energy delivery;
a cylindrical distal tip (3) joined to said tubular body (2), said cylindrical distal tip (3) defining at least one internal needle director channel (17a'), each of said needle director channels (17a') communicating with one of said at least one needle lumens (16), and opening via a needle outlet (18a) onto the periphery of said cylindrical distal tip (3);
track means (27) for receiving and guiding said bearing (26) in said cylindrical distal tip (3), said track means (27) being configured to guide said sharpened distal end (34) of said rigid distal needle portion (15a) out of said needle outlet (18a) when said flexible control and actuation cable (25) is moved distally; and
means (28, 29, 30, 31) for selectively locking said rigid needle portion (15a) with respect to said tubular body (2), to provide support for said at least one needle means (15) when puncturing said passageway wall and organ (43), in a position extending out of said needle outlet (18a).

15. The apparatus according to claim 14, wherein said means (28, 29, 30, 31) for selectively locking said rigid distal needle portion (15a) with respect to said tubular body (2) in a position extending out of said needle outlet (18a) is further characterized in that it includes:
a catch (28);
a spring (29);
a pivot (30); and
a cable (31);
such that in order to finally position said at least one needle means (15), said catch (28) is retracted by said cable (31); and said bearing (26) is pushed past said catch (28); with said cable (31) then being released and spring (29) causing said catch (28) to hold said at least one needle means (15) in place due to its biasing action around said pivot (30).

16. The apparatus according to claim 14, further characterized in that said rigid needle portion (15a) is stainless steel and said sharpened distal end (34) has a razor-sharp edge.

17. The apparatus according to claim 5, still further characterized in that there are five fiber optic elements (23'), five tubular shaft needles (15'), five needle lumens (16') and five curved needle director channels (17').

18. The apparatus according to claim 5, still further characterized in that there are five fiber optic elements (23') and five tubular shaft needles (15'), received in a single needle lumen (16') communicating with five curved needle director channels (17'), which individually receive each of said tubular shaft needles (15').

19. The apparatus according to claim 1, further characterized in that said body passageway (42) is one selected from the group consisting of the urethra and the rectum; and said organ (43) to be treated is the prostate.

## Patentansprüche

1. Ein Gerät zur Laserenergielieferung an ein Organ (43), das an einen durch eine Kanalwand begrenzten Körperkanal (42) angrenzt, wobei das Gerät umfaßt:
wenigstens ein Faseroptikelement (23) zum Liefern von Laserlicht von einer Laserenergiequelle (11) an einen Bereich des zu behandelnden Organs (43);
wenigstens eine Nadeleinrichtung (15) zum mechanischen Punktieren der Kanalwand und des Organs (43);
eine Nadel- und Faseroptikelement-Beförderungseinrichtung (1) zum Befördern jeder der wenigstens einen Nadeleinrichtungen (15) und jeder der wenigstens einen Faseroptikelemente (23) bis zu einer Position im Kanal (42) angrenzend an das zu behandelnde Organ (43);
wobei die Nadel- und Faseroptikelement-Beförderungseinrichtung (1) ein rohrförmiger Körper (2) mit einer äußeren Umfangsfläche, einem distalen Ende (4) und einem proximalen Ende (5) ist und wenigstens einen Innenkanal (16) für die Nadeln entsprechend jeder der wenigstens einen Nadeleinrichtungen (15) zum gleitenden Aufnehmen der wenigstens einen Nadeleinrichtungen (15) begrenzt;
wobei die Nadel- und Faseroptikelement-Befördervngseinrichtung (1) weiterhin wenigstens eine Nadelleiteinrichtung (17, 18) zum Leiten der wenigstens einen Nadeleinrichtung (15) in das Organ (43) umfaßt, und die Nadelleiteinrichtung (17, 18) wenigstens einen gekurvten Nadelleitkanal (17) so umfaßt, daß dieser eine gekurvte Nadelleitkanal (17) mit jedem der wenigstens einen Innenkanäle (16) für die Nadeln kommuniziert und einen Ausgang (18) auf der Außenfläche des rohrförmigen Körpers (2) hat, der sich in den Körperkanal (42) öffnet;
und jede der wenigstens einen Nadeleinrichtungen (15) zum mechanischen Punktieren der Kanalwand und des Organs (43) einen flexiblen rohrförmigen Schaft (33) und eine angeschärfte distale Nadelspitze (34) umfaßt;
dadurch gekennzeichnet, daß:
das wenigstens eine Faseroptikelement (23) ein unversperrtes distales Ende aufweist;
jede der wenigstens einen Nadeleinrichtungen (15) hohl ist und in sich einen Innenkanal (32) begrenzt, der eine unversperrte distale Öffnung (44) aufweist und dem Befördern eines der wenigstens einen Faseroptikelemente (23) durch die Punktierstelle zum Bereich des zu behandelnden Organs (43) dient; und
jedes der wenigstens einen Faseroptikelemente (23) gleitfähig innerhalb einer der wenigstens einen Nadeleinrichtungen (15) angeordnet ist zur Ermöglichung einer relativen axialen Bewegung zwischen dem wenigstens einen Faseroptikelement (23) und der wenigstens einen Nadeleinrichtung (15) so, daß das wenigstens eine Faseroptikelement (23) aus der distalen Öffnung (44) des Innenkanals (32) vorschiebbar ist und jedes der wenigstens einen Nadeleinrichtung (15) separat vom darin gleitfähig angeordneten Faseroptikelement (23) zurückziehbar ist, zur Freilegung des unversperrten distalen Endes des Faseroptikelements (23) gegenüber dem zu behandelnden Bereich.

2. Gerät nach Anspruch 1, weiterhin dadurch gekennzeichnet, daß jede der wenigstens einen Nadeleinrichtungen (15) individuell lenkbar ist.

3. Gerät nach Anspruch 2, weiterhin dadurch gekennzeichnet, daß jede der wenigstens einen Nadeleinrichtungen (15) weiterhin umfaßt:
eine periphere Öffnung (40) im flexiblen rohrförmigen Nadelschaft (33), die von der distalen Spitze (34) der Nadeleinrichtung (15) in Proximalrichtung einen Abstand aufweist; und
eine im Innenkanal (32) in jeder der wenigstens einen Nadeleinrichtungen (15) aufgenommene Lenkfaser (39), die den Innenkanal (32) durch die periphere Öffnung (40) verläßt und mit der Nadeleinrichtung (15) angrenzend an ihre metallene angeschärfte distale Spitze (34) an einer Verbindung (41) verbunden ist, wodurch die Nadeleinrichtung (15) durch das Einführen einer Spannung in die Lenkfaser (39) biegbar und lenkbar ist.

4. Gerät nach Anspruch 1, weiterhin dadurch gekennzeichnet, daß es weiterhin eine Absaug-Irrigations-Einrichtung (12) zum alternativen Absaugen und Irrigieren des Körperkanals (42) um die Punktierstelle nach Wunsch umfaßt derart, daß die Absaug-Irrigations-Einrichtung (12) mit einem der folgenden Räume kommuniziert: mit jedem der Nadel-Innenkanäle (16), und mit jedem der Innenkanäle (32) in jeder der wenigsten einen Nadeleinrichtungen (15).

5. Gerät nach Anspruch 1, weiterhin dadurch gekennzeichnet, daß:
die wenigstens eine Nadeleinrichtung (15) eine Anzahl von einzelnen rohrförmigen Schaftnadeln (15') enthält, von denen jede einzelne rohrförmige Schaftnadel (15') einen flexiblen Schaft (33') und eine angeschärfte distale Spitze (34') zum Punktieren der Kanalwand und des Organs (43) aufweist und einen Innenkanal (32') zum gleitfähigen Aufnehmen eines entsprechenden aus einer gleichen Anzahl einzelner Faseroptikelemente (23') begrenzt, die das wenigstens eine Faseroptikelement (23) bilden;
die Nadel- und Faseroptikelement-Beförderungseinrichtung (1) ein rohrförmiger Körper (2) mit einem proximalen Ende (5) und einem distalen Ende (4) ist und eine Mehrzahl von Innenkanälen (16') für Nadeln in einer Anzahl gleich der Anzahl der einzelnen rohrförmigen Schaftnadeln (15') begrenzt, wobei in jedem Innenkanal (16') für die Nadeln eine rohrförmige Schaftnadel (15') gleitfähig aufgenommen ist;
eine eine Außenfläche aufweisende zylindrische distale Spitze (3) am distalen Ende (4) des rohrförmigen Körpers (2) befestigt ist und eine Mehrzahl von gekurvten Nadelleitkanälen (17') zahlenmäßig gleich der Anzahl der Innenkanäle (16') für die Nadeln aufweist, wobei jeder kurvige Nadelleitkanal (17') mit einem Innenkanal (16') für die Nadeln am distalen Ende (4) des rohrförmigen Körpers (2) kommuniziert und sich an einem Nadelausgang (18') entlang der Außenfläche der distalen Spitze (3) öffnet zum Schaffen einer Einrichtung zum Lenken der rohrförmigen Schaftnadeln (15') in das Organ (43).

6. Gerät nach Anspruch 1, weiterhin mit einer Fixiereinrichtung zum vorübergehenden Fixieren des rohrförmigen Körpers (2) und der distalen Spitze (3) im Körperkanal (42) relativ zur Kanalwand, wobei die Fixiereinrichtung umfaßt:
einen ersten aufpumpbaren Dehnungsballon 20), der den rohrförmigen Körper (2) umfangsmäßig umgibt;
einen Aufpump-Innenkanal (22) im rohrförmigen Körper (2), der mit dem ersten aufpumpbaren Dehnungsballon (20) kommuniziert;
einen zweiten aufpumpbaren Dehnungsballon (19), der am distalen Ende der distalen Spitze (3) angeordnet ist; und
einen im rohrförmigen Körper (2) verlaufenden Aufpump-Innenkanal (21), der durch die distale Spitze (3) bis in Kommunikation mit dem zweiten aufpumpbaren Dehnungsballon verlängert ist;
weiterhin dadurch gekennzeichnet, daß:
der erste aufpumpbare Dehnungsballon (20) proximal relativ zu den Nadelausgängen (18) in der distalen Spitze (3) angeordnet ist; und
der erste aufpumpbare Dehnungballon (20) und der zweite aufpumpbare Dehnungsballon (19) beide gemeinsam an einem einzigen Gerät vorhanden sind und unabhängig aufpumpbar und entleerbar sind.

7. Gerät nach Anspruch 1, weiterhin mit einer Fixiereinrichtung zum vorübergehenden Fixieren des rohrförmigen Körpers (2) und der distalen Spitze (3) im Körperkanal (42) relativ zur Kanalwand, wobei die Fixiereinrichtung umfaßt:
einen aufblasbaren Dehnungsballon (19a);
einen Aufpump-Innenkanal (21), der vom rohrförmigen Körper (2) begrenzt ist und mit dem aufpumpbaren Dehnungsballon (19a) kommuniziert;
weiterhin dadurch gekennzeichnet, daß:
der aufpumpbare Dehnungsballon (19a) die distale Spitze (3) und wenigstens einen Teil des distalen Endes (4) des rohrförmigen Körpers (2) umgibt; und
der aufpumpbare Dehnungsballon (19a) eine Anzahl von Nadelkanälen (17'') gleich der Zahl der Innenkanäle (16'') für die Nadeln umfaßt und die Nadeln durch diese Nadelkanäle hindurchtreten können, von denen jeder mit einem der Nadelauslässe (18'') fluchtet und kommuniziert.

8. Gerät nach Anspruch 1, weiterhin dadurch gekennzeichnet, daß jedes der wenigstens einen Faseroptikelemente (23) eine Lichtleitfaser (36), die von einer optischen Beschichtung (38) umgeben ist, mit einer koaxial zur Lichtleitfaser (36) auf der optischen Beschichtung (38) vorgesehenen Wärmemeßvorrichtung (24) einschließt.

9. Gerät nach Anspruch 8, weiterhin dadurch gekennzeichnet, daß die Lichtleitfaser (36) ein sphärisches distales Ende (37) aufweist.

10. Gerät nach Anspruch 1, weiterhin dadurch gekennzeichnet, daß das Faseroptikelement (23) eine blanke Lichtleitfaser mit wenigstens einer Wärmemeßvorrichtung (24') daran besteht.

11. Gerät nach Anspruch 10, weiterhin dadurch gekennzeichnet, daß das Faseroptikelement (23) ein sphärisches distales Ende (37') aufweist.

12. Gerät nach Anspruch 1, weiterhin gekennzeichnet durch die Einbeziehung einer Nadelbetätigungs- und -steuereinrichtung (8) für die individuelle Betätigung und Steuerung des wenigstens einen Nadel-Innenkanals (16) zum Bewegen der Nadeleinrichtung (15) zwischen einer ersten Stellung, in der sie sich hülsenartig innerhalb des wenigstens einen Nadel-Innenkanals (16) befindet, und einer zweiten Stellung, in der sie aus dem rohrförmigen Körper (2) heraus und in das zu behandelnde Organ (43) hinein vorsteht, wobei die Nadelbetätigungs- und -steuereinrichtung (8) in der Lage ist, die wenigstens eine Nadeleinrichtung (15) alternativ zwischen der ersten Stellung und der zweiten Stellung sowohl unabhängig von dem wenigstens einen Faseroptikelement (23) als auch in Verbindung mit dem wenigstens einen Faseroptikelement (23) zu bewegen, wodurch das wenigstens eine Faseroptikelement (23) und die wenigstens eine Nadeleinrichtung (15) und Organ (43) nach Bedarf unabhängig selektiv positioniert und neu positioniert werden können.

13. Gerät nach Anspruch 12, weiterhin dadurch gekennzeichnet, daß die wenigstens eine Nadeleinrichtung (15) durch Mittel betätigt ist, die gewählt sind aus der Gruppe bestehend aus hydraulischen Mitteln, federgeladenen Mitteln, einem mechanischen querverschieblichen Kabel, einem mechanischen Drehkabel, elektromagnetischen Mitteln und einer Formerinnerungslegierung.

14. Gerät nach Anspruch 1, weiterhin dadurch gekennzeichnet, daß:
jede der wenigstens einen Nadeleinrichtungen (15) einen starren distalen Nadelteil (15a) mit einem proximalen Ende (15b) und einem angeschärften distalen Ende (34) umfaßt, wobei der starre distale Nadelteil (15a) einen hohlen Innenkanal (32') zum gleitfähigen Tragen des Faseroptikelements begrenzt; und
die Nadel- und Faseroptikelement-Beförderungseinrichtung (1) weiterhin einen rohrförmigen Körper (2) enthält, der wenigstens einen Nadel-Innenkanal (16) begrenzt; und
das Gerät weiterhin umfaßt:
ein auf dem starren distalen Nadelteil (15a) an seinem proximalen Ende 15b) montiertes Lager (26);
wenigstens ein flexibles Steuer- und Betätigungs-Kabel (25), das am proximalen Ende (15b) des starren Teils (15a) jeder der wenigstens einen Nadeleinrichtungen (15) befestigt ist und gleitfähig im wenigstens einen Nadel-Innenkanal (16) des rohrförmigen Körpers (2) angeordnet ist;
einen Aufbau (33'), der am proximalen Ende (15b) des starren distalen Nadelteils (15a) befestigt ist und wenigstens einen Innenkanal (32'') begrenzt, wobei der Innenkanal (32'') mit dem hohlen Innenkanal (32') kommuniziert, gleitfähig eines der wenigstens einen Faseroptikelemente (23) trägt und eine Einrichtung zum Absaugen und Irrigieren des Bereichs der Energielieferung bildet;
eine sich an den rohrförmigen Körper (2) anschließende zylindrische distale Spitze (3), die wenigstens einen internen Nadelleitkanal (17a') begrenzt, von denen jeder mit einem der Nadel-Innenkanäle (16) kommuniziert, und der sich über einen Nadelausgang (18a) zur Außenfläche der zylindrischen distalen Spitze öffnet;
Führungsspureinrichtungen (27) zum Aufnehmen und Führen des Lagers (26) in der zylindrischen distalen Spitze (3), wobei die Führungsspureinrichtungen (27) so gestaltet sind, daß sie das angeschärfte distale Ende (34) des starren distalen Nadelteils (15a) aus dem Nadelausgang (18a) herausführen, wenn das flexible Steuer- und Betätigungs-Kabel (25) distal bewegt wird; und
eine Einrichtung (28, 29, 30, 31) zum selektiven Verriegeln des starren Nadelteils (15a) in Bezug zum rohrförmigen Körper (2) in einer aus dem Nadelausgang (18a) vorstehenden Stellung, zum Schaffen einer Abstützung für die wenigstens eine Nadeleinrichtung (15) beim Punktieren der Kanalwand und des Organs (43).

15. Gerät nach Anspruch 14, bei dem die Einrichtung (28, 29, 30, 31) zum selektiven Verriegeln des starren distalen Nadelteils (15a) in Bezug zum rohrförmigen Körper (2) in einer aus dem Nadelausgang (18a) vorstehenden Stellung weiterhin dadurch gekennzeichnet ist, daß sie enthält:
einen Mitnehmer (28);
eine Feder (29);
einen Drehzapfen (30); und
ein Kabel (31);
derart, daß: zum schließlichen Positionieren der wenigstens einen Nadeleinrichtung (15) der Mitnehmer (28) durch das Kabel (31) zurückgezogen wird; das Lager (26) am Mitnehmer (28) vorbeigeschoben wird; woraufhin das Kabel (31) entspannt wird und die Feder (29) bewirkt, daß der Mitnehmer (28) die wenigstens eine Nadeleinrichtung (15) aufgrund ihrer vorspannenden Wirkung um den Drehzapfen (30) an Ort und Stelle hält.

16. Gerät nach Anspruch 14, weiterhin dadurch gekennzeichnet, daß der starre Nadelteil (15a) aus rostfreiem Stahl besteht und das angescharrte distale Ende (34) einen rasierklingenscharfen Rand aufweist.

17. Gerät nach Anspruch 5, weiterhin dadurch gekennzeichnet, daß fünf Faseroptikelemente (23'), fünf rohrförmige Schaftnadeln (15'), fünf Innenkanäle (16') für die Nadeln und fünf Leitkanäle (17') für kurvige Nadeln vorhanden sind.

18. Gerät nach Anspruch 5, weiterhin dadurch gekennzeichnet, daß fünf Faseroptikelemente (23') und fünf rohrförmige Schaftnadeln (15'), die in einem einzigen Innenkanal (16') für die Nadeln enthalten sind, der mit fünf Leitkanälen (17') für kurvige Nadeln zum individuellen Aufnehmen jeder der rohrförmigen Schaftnadeln (15') kommuniziert, vorhanden sind.

19. Gerät nach Anspruch 1, weiterhin dadurch gekennzeichnet, daß der Körperkanal (42) die Uretra oder das Rektum ist; und daß das zu behandelnde Organ (43) die Prostata ist.

## Revendications

1. Appareil pour envoyer une énergie laser sur un organe (43) au voisinage d'un passage corporel (42) délimité par une cloison, ledit appareil comprenant :
au moins un élément de fibre optique (23) destiné à diriger une lumière laser d'une source d'énergie laser (11) sur une zone de l'organe (43) devant être traitée ;
au moins un moyen à aiguille (15) pour perforer mécaniquement la cloison du passage et l'organe (43) ;
un moyen (1) d'amenée d'aiguille et d'élément de fibre optique afin de placer chacun desdits au moins un moyen d'aiguille (15) et chacun desdits au moins un élément de fibre optique (23) à une position située dans le passage (42) au voisinage de l'organe (43) devant être traité ;
ledit moyen (1) d'amenée d'aiguille et d'élément de fibre optique étant un corps tubulaire (2) ayant une périphérie extérieure, une extrémité distale (4) et une extrémité proximale (5) et délimitant au moins un passage d'aiguille (16) correspondant à chacun desdits au moins un moyen d'aiguille (15) afin de loger à coulissement ledit au moins un moyen à aiguille (15) ;
ledit moyen (1) d'amenée d'aiguille et d'élément de fibre optique comprenant par ailleurs au moins un moyen d'orientation d'aiguille (17, 18) pour diriger ledit au moins un moyen à aiguille (15) dans l'organe (43), ledit moyen d'orientation d'aiguille (17, 18) comprenant au moins un canal courbe (17) d'orientation de l'aiguille de façon que ledit un canal courbe (17) d'orientation d'aiguille communique avec chacun desdits au moins un passage d'aiguille (16), et comprenant à la périphérie dudit corps tubulaire (2) une sortie (18) s'ouvrant sur ledit passage corporel (42) ; et
chacun desdits au moins un moyen à aiguille (15) destiné à perforer mécaniquement la cloison du passage et l'organe (43) comprenant une tige tubulaire souple (33) et un bout effilé distal d'aiguille (34) relié par un joint (35) à ladite tige (33),
caractérisé en ce que
ledit au moins un élément de fibre optique (23) comporte une extrémité distale inobstruée ;
chacun desdits au moins un moyen à aiguille (15) est creux et délimite intérieurement un passage (32) comportant une ouverture distale inobstruée (44), ledit passage (32) étant destiné à transporter l'un desdits au moins un élément de fibre optique (23) par la perforation vers la zone de l'organe (43) devant être traitée ; et
chacun desdits au moins un élément de fibre optique (23) est disposé coulissant à l'intérieur de l'un desdits au moins un moyen à aiguille (15) pour permettre un mouvement axial relatif entre ledit au moins un élément de fibre optique (23) et ledit au moins un moyen à aiguille (15) de façon que ledit au moins un élément de fibre optique (23) puisse être déplacé au-delà de ladite ouverture distale (44) dudit passage (32), chacun desdits au moins un moyen à aiguille (15) pouvant être mis séparément en retrait par rapport audit élément de fibre optique (23) qui est disposé coulissant en elle afin d'exposer ladite extrémité distale inobstruée dudit élément de fibre optique (23) à la zone devant être traitée.

2. Appareil selon la revendication 1, caractérisé par ailleurs en ce que chacun desdits au moins un moyen à aiguille (15) peut être guidé individuellement.

3. Appareil selon la revendication 2, caractérisé par ailleurs en ce que chacun desdits au moins un moyen à aiguille (15) comprend par ailleurs :
une ouverture périphérique (40) réalisée dans ladite tige tubulaire souple d'aiguille (33) et placée du côté proximal par rapport au bout distal (34) dudit moyen à aiguille (15) ; et une fibre de guidage (39) disposée dans ledit passage (32) réalisé dans chacun desdits au moins un moyen à aiguille (15) et sortant dudit passage (32) par ladite ouverture périphérique (40), ladite fibre de guidage (39) étant reliée par un joint (41) audit moyen à aiguille (15) au voisinage de sa pointe distale métallique effilée d'aiguille (34), de façon que ledit moyen à aiguille (15) puisse être courbé et guidé par l'exercice d'une traction dans ladite fibre de guidage (39).

4. Appareil selon la revendication 1, caractérisé par ailleurs en ce que ledit appareil comprend en outre un moyen d'aspiration-irrigation (12) pour effectuer en alternance une aspiration et une irrigation dudit passage (42) situé autour de la perforation, si cela est souhaité, de façon que ledit moyen d'aspiration-irrigation (12) communique avec au moins l'un : de chacun desdits passages d'aiguille (16) et de chacun desdits passages (32) situés dans chacun desdits au moins un moyen à aiguille (15).

5. Appareil selon la revendication 1, caractérisé par ailleurs en ce que :
ledit au moins un moyen à aiguille (15) comprend plusieurs aiguilles individuelles à tige tubulaire (15'), chacune desdites aiguilles individuelles à tige tubulaire (15') comprenant une tige souple (33') et un bout distal effilé (34') pour perforer ladite paroi du passage et l'organe (43) et délimitant un passage (32') destiné à loger à coulissement l'un correspondant d'un nombre égal d'éléments individuels de fibres optiques (23') constituant ledit au moins un élément de fibre optique (23) ;
ledit moyen (1) d'amenée d'aiguille et d'élément de fibre optique étant un corps tubulaire (2) ayant une extrémité proximale (5) et une extrémité distale (4) et délimitant plusieurs passages d'aiguille (16') dont le nombre est égal au nombre desdites aiguilles individuelles à tige tubulaire (15'), une aiguille à tige tubulaire (15') étant logée coulissante dans chacun desdits passages d'aiguille (16') ;
un bout cylindrique distal (3) comportant une périphérie extérieure étant fixé à l'extrémité distale (4) du corps tubulaire (2), ledit bout distal (3) comportant plusieurs canaux courbes d'orientation d'aiguille (17') dont le nombre est égal au nombre des passages d'aiguille (16'), chaque canal courbe d'orientation d'aiguille (17') communiquant avec un passage d'aiguille (16') à l'extrémité distale (4) du corps tubulaire (2) et s'ouvrant sur une sortie d'aiguille (18') le long de la périphérie du bout distal (3) afin de constituer un moyen pour diriger lesdites aiguilles (15') à tige tubulaire dans l'organe (43).

6. Appareil selon la revendication 1, comprenant par ailleurs un moyen de fixation pour fixer temporairement ledit corps tubulaire (2) et ledit bout distal (3) dans ledit passage corporel (42) par rapport à ladite cloison du passage ;
ledit moyen de fixation comprenant :
un premier ballonnet gonflable de dilatation (20) entourant à la circonférence ledit corps tubulaire (2) ;
un passage de gonflage (22) réalisé dans ledit corps tubulaire (2) et communiquant avec ledit premier ballonnet gonflable de dilatation (20) ;
un second ballonnet gonflable de dilatation (19) disposé à l'extrémité distale du bout distal (3) ; et
un passage de gonflage (21) réalisé dans ledit corps tubulaire (2) et passant à travers ledit bout distal (3) de manière à communiquer avec ledit second ballonnet gonflable de dilatation (19) ;
caractérisé par ailleurs en ce que :
ledit premier ballonnet gonflable de dilatation (20) est disposé du côté proximal par rapport auxdites sorties d'aiguille (18) situées dans ledit bout distal (3) ; et
ledit premier ballonnet gonflable de dilatation (20) et ledit second ballonnet gonflable de dilatation (19) sont tous deux placés ensemble sur un unique appareil et sont capables d'être indépendamment gonflés et dégonflés.

7. Appareil selon la revendication 1, comprenant par ailleurs un moyen de fixation pour fixer temporairement le corps tubulaire (2) et ledit bout distal (3) dans ledit passage corporel (42) par rapport à ladite cloison de ce passage, ledit moyen de fixation comprenant :
un ballonnet gonflable de dilatation (19a) ;
un passage de gonflage (21) délimité par ledit corps tubulaire (2) et communiquant avec ledit ballonnet gonflable de dilatation (19a) ;
caractérisé par ailleurs en ce que :
ledit ballonnet gonflable de dilatation (19a) entoure ledit bout distal (3) et au moins une partie de ladite extrémité distale (4) dudit corps tubulaire (2) ; et
ledit ballonnet gonflable de dilatation (19a) comporte plusieurs canaux d'aiguille (17'') dont le nombre est égal à celui des passages d'aiguille (16'') par lesquels lesdites aiguilles peuvent passer, chacun desdits canaux d'aiguille (17'') étant à l'alignement de, et communiquant avec, l'une desdites sorties (18'') d'aiguille.

8. Appareil selon la revendication 1, caractérisé par ailleurs en ce que chacun desdits au moins un élément de fibre optique (23) comprend une fibre optique (36) entourée d'un revêtement optique (38), un dispositif de thermo-mesure (24) étant placé coaxialement à ladite fibre optique (36) sur ledit revêtement optique (38).

9. Appareil selon la revendication 8, caractérisé par ailleurs en ce que ladite fibre optique (36) comporte une extrémité distale sphérique (37).

10. Appareil selon la revendication 1, caractérisé par ailleurs en ce que ledit élément de fibre optique (23) est une fibre optique nue sur laquelle au moins un dispositif de thermo-mesure (24') est placé.

11. Appareil selon la revendication 10, caractérisé par ailleurs en ce que ledit élément de fibre optique (23) comporte une extrémité distale sphérique (37').

12. Appareil selon la revendication 1, caractérisé par ailleurs par l'inclusion d'un moyen (8) d'actionnement et de commande d'aiguille pour actionner et commander individuellement ledit au moins un passage d'aiguille (16) afin de déplacer ledit moyen à aiguille (15) entre une première position, à laquelle il est gainé dans ledit au moins un passage d'aiguille (16), et une seconde position à laquelle il se prolonge à l'extérieur dudit corps tubulaire (2) et dans ledit organe (43) devant être traité, ledit moyen (8) d'actionnement et de commande d'aiguille étant capable de déplacer ledit au moins un moyen à aiguille (15) entre lesdites première et seconde positions, en alternance, indépendamment dudit au moins un élément de fibre optique (23) et conjointement avec ledit au moins un élément de fibre optique (23), de manière que ledit au moins un élément de fibre optique (23) et ledit au moins un moyen à aiguille (15) soient capables d'être positionnés et repositionnés sélectivement et indépendamment dans ledit organe (43), de la manière qui est nécessaire.

13. Appareil selon la revendication 12, caractérisé par ailleurs en ce que l'actionnement dudit au moins un moyen à aiguille (15) s'effectue par un moyen choisi dans le groupe consistant en un câble hydraulique, un câble chargé par ressort, un câble à translation mécanique, un câble rotatif mécaniquement, un alliage électromagnétique et un alliage à mémoire de forme.

14. Appareil selon la revendication 1, caractérisé par ailleurs en ce que :
chacun desdits au moins un moyen à aiguille (15) comprend une partie distale rigide d'aiguille (15a) ayant une extrémité proximale (15b) et une extrémité distale effilée (34), ladite partie distale rigide d'aiguille (15a) comportant un passage (32') destiné à supporter ledit élément de fibre optique de manière qu'il soit coulissant ; et
ledit moyen (1) d'amenée d'aiguille et d'élément de fibre optique comprend par ailleurs un corps tubulaire (2) délimitant au moins un passage d'aiguille (16) ; et
ledit appareil comprenant par ailleurs :
une portée (26) montée sur ladite partie distale rigide d'aiguille (15a) à son extrémité proximale (15b) ;
au moins un câble souple de commande et d'actionnement (25) fixé à ladite extrémité proximale (15b) de ladite partie rigide (15a) de chacun desdits au moins un moyen à aiguille (15) et disposé coulissant à l'intérieur dudit au moins un passage d'aiguille (16) dudit corps tubulaire (2) ;
une structure (33') fixée à ladite extrémité proximale (15b) de ladite partie distale rigide d'aiguille (15a) et délimitant au moins un passage (32''), ledit passage (32'') communiquant avec ledit passage (32'), supportant coulissant l'un desdits au moins un élément de fibre optique (23) et constituant un moyen pour aspirer et irriguer la zone sur laquelle l'énergie est envoyée ;
un bout cylindrique distal (3) relié audit corps tubulaire (2), ledit bout distal cylindrique (3) délimitant au moins un canal interne d'orientation d'aiguille (17a'), chacun desdits canaux d'orientation d'aiguille (17a') communiquant avec l'un desdits au moins un passage d'aiguille (16) et s'ouvrant par une sortie d'aiguille (18a) dans la périphérie dudit bout cylindrique distal (3) ;
un moyen de voie (27) pour recevoir et guider ladite portée (26) dans ledit bout distal cylindrique (3), ledit moyen de voie (27) étant conformé pour guider ladite extrémité distale effilée (34) de ladite partie distale rigide d'aiguille (15a) vers l'extérieur de ladite sortie d'aiguille (18a) lorsque ledit câble souple de commande et d'actionnement (25) est déplacé vers le côté distal ; et
des moyens (28, 29, 30, 31) pour bloquer sélectivement ladite partie rigide d'aiguille (15a) par rapport audit corps tubulaire (2) afin d'assurer un support dudit au moins un moyen à aiguille (15) lors de la perforation de ladite paroi du passage et de l'organe (43) à une position située à l'extérieur de ladite sortie d'aiguille (18a).

15. Appareil selon la revendication 14, dans lequel lesdits moyens (28, 29, 30, 31) destinés à bloquer sélectivement ladite partie distale rigide d'aiguille (15a) par rapport audit corps tubulaire (2) à une position située à l'extérieur de ladite sortie d'aiguille (18a) sont par ailleurs caractérisés en ce qu'ils comprennent :
un loquet (28) ;
un ressort (29) ;
un pivot (30) ; et
un câble (31) ;
de façon qu'afin de mettre à la position finale ledit au moins un moyen à aiguille (15), le loquet (28) soit rétracté par ledit câble (31) ; et ladite portée (26) soit poussée au-delà dudit loquet (28) ; ledit câble (31) étant alors libéré et le ressort (29) faisant en sorte que ledit loquet (28) tienne ledit au moins un moyen à aiguille (15) en place par suite de son effet de sollicitation autour dudit pivot (30).

16. Appareil selon la revendication 14, caractérisé par ailleurs en ce que ladite partie rigide d'aiguille (15a) est en acier inoxydable et ladite extrémité distale effilée (34) comporte un bord ayant le fil d'un rasoir.

17. Appareil selon la revendication 5, caractérisé par ailleurs en ce qu'il y a cinq éléments de fibre optique (23'), cinq aiguilles à tige tubulaire (15'), cinq passages d'aiguille (16') et cinq canaux courbes (17') d'orientation d'aiguille.

18. Appareil selon la revendication 5, caractérisé par ailleurs en ce qu'il y a cinq éléments de fibre optique (23') et cinq aiguilles (15') à tige tubulaire logés dans un unique passage d'aiguille (16') communiquant avec cinq canaux courbes (17') d'orientation d'aiguille qui logent individuellement chacune desdites aiguilles à tiges tubulaires (15').

19. Appareil selon la revendication 1, caractérisé par ailleurs en ce que ledit passage corporel (42) est l'un choisi dans le groupe consistant en l'urètre et le rectum ; et ledit organe (43) devant être traité est la prostate.
